(19) Europäisches Patentamt
European Patent Office
Office européen des brevets

(11) Veröffentlichungsnummer: **0 227 007**
**A2**

(12) # EUROPÄISCHE PATENTANMELDUNG

(21) Anmeldenummer: **86117482.9**

(51) Int. Cl.4: **C07K 5/06**

(22) Anmeldetag: **16.12.86**

(30) Priorität: **20.12.85 DE 3545193**

(43) Veröffentlichungstag der Anmeldung:
**01.07.87 Patentblatt 87/27**

(84) Benannte Vertragsstaaten:
**BE CH DE FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Schlingmann, Merten, Dr.**
**Schneidhainer Strasse 32a**
**D-6240 Königstein/Taunus(DE)**
Erfinder: **Keller, Reinhold, Dr.**
**Wiesenweg 5**
**D-6232 Bad Soden am Taunus(DE)**
Erfinder: **Dürsch, Walter, Dr.**
**In der Braubach 4**
**D-6240 Königstein/Taunus(DE)**
Erfinder: **Hoppe, Hans-Ullrich, Dr.**
**Habichtsweg 4**
**D-6238 Hofheim am Taunus(DE)**

(54) **N-(4-Benzyl-L-alpha-aspartyl)-L-phenylalaninmethylester, Verfahren zu seiner Herstellung und seine Verwendung.**

(57) N-(4-Benzyl-L-d-aspartyl)-L-phenylalaninmethylester, ein Zwischenprodukt bei der Herstellung von Aspartam, kann durch Umsetzung von 4-Benzyl-L-aspartat-N-carbonsäureanhydrid mit Phenylalaninmethylester in wäßriger Lösung bei pH-Werten von 6,0 bis 8,5 in einem einfachen Verfahren hergestellt werden.

EP 0 227 007 A2

## N-(4-Benzyl-L-d-aspartyl)-L-phenylalaninmethylester, Verfahren zu seiner Herstellung und seine Verwendung

N-(4-Benzyl-L-aspartyl)-L-phenylalaninmethylester ("Benzyl-aspartam", Formel I) ist von Interesse als Vorstufe für die Herstellung des Süßstoffs L-α-Aspartyl-L-phenylalaninmethylester ("Aspartam"). Aspartam kann aus Benzylaspartam einfach nach bekannten Methoden durch hydrogenolytische Abspaltung des Benzylrestes erhalten werden.

Es wurde bereits ein Verfahren beschrieben, bei dem allgemein ein α-Aminocarbonsäureester mit einem α-Aminocarbonsäure-N-carbonsäure-anhydrid und einem tertiären Amin in organischen Lösungsmitteln umgesetzt wird und zuerst das Trialkylammoniumcarbaminat des Peptidesters und dann der α-Peptidester erhalten wird [J.Chem. Soc. 3461, (1950) und J.prak.Ch.4, 261 (1955)]. Diese Methode hat folgende Nachteile:

1. Es müssen tiefe Temperaturen, von beispielsweise -30°C und darunter, eingehalten werden.

2. Die α-Aminosäureester müssen unbedingt, in freier basischer Form isoliert, dem Reaktionsgemisch zugegeben werden.

3. Die verwendeten tertiären Amine müssen aufgearbeitet werden.

Die Herstellung von Benzylaspartam aus 4-Benzyl-L-aspartat-N-carbonsäure-anhydrid (Formel II) und L-Phenylalaninmethylester in wäßriger Lösung bei pH-Werten von 9 bis 12 wurde in der inzwischen zurückgezogenen japanischen Patentanmeldung 28042/1972 beansprucht, von der sonst keine ausländischen Äquivalenzen existieren. Die dort in Beispiel 1 aufgeführten Reaktionsparameter basieren auf einem angeblichen 4-Benzyl-L-aspartat-N-carbonsäure-anhydrid, das nur einen Fp. von 66,5° C aufwies. Der tatsächliche Fp. der reinen Verbindung liegt jedoch wesentlich höher, nämlich bei 121°C gemäß J.Am.Chem.Soc. 73,4087, (1951), bei 127-128°C (unter Zersetzung) nach eigenen Befunden.
Schon deswegen muß die im Beispiel 1 der japanischen Anmeldung angegebene Ausbeute von 92 % d.Th. an rohem Benzylaspartam, die bei einem pH-Wert von 10,2 erhalten worden sein soll, angezweifelt werden. Ferner geht aus einem Artikel in J.Am.Chem.Soc. 93,2746, (1971), der wiederum auf J.Org.Chem. 32, S. 3421 (1967) aufbaut, hervor, daß bei pH 10,2 und sehr kurzen Reaktionszeiten von nur 15 bis 30 Sekunden die Alkali-Salze von α-Aminocarbonsäuren im allgemeinen mit estergruppenfreien α-Aminocarbonsäure-N-carbonsäure-anhydriden ("Leuchs-schen Anhydriden") zu den entsprechenden α-Dipeptiden umgesetzt werden können. Es wird in demselben Artikel jedoch auch festgestellt, daß 4-Benzyl-L-aspartatN-carbonsäure-anhydrid bei derart hohen pH-Werten, selbst bei den extrem kurzen Reaktionszeiten, nur unter Verlust des 4-Benzylrestes zu verschiedenen Aspartyl-dipeptiden umgesetzt werden konnte. Leider kann das Beispiel 1 der japanischen Anmeldung, wegen ungenauer Versuchsdaten, wie "allmähliche Zugabe" des gepulverten angeblichen N-Carbonsäure-anhydrid bei pH 10,2 und fehlender Angaben über die verwendeten Mengen von Natriumborat-bzw. Salpetersäurelösung nicht exakt nachgearbeitet und überprüft werden.

Es wurde nun überraschend eine sehr einfaches Verfahren zur Herstellung von Benzylaspartam (Formel I) gefunden: Man setzt 4-Benzyl-L-aspartat-N-carbonsäure-anhydrid (Formel II) und L-Phenylalaninmethylester in wäßriger Lösung bei pH-Werten von 6,0 bis 8,5 um und isoliert das entstandene Benzylaspartam nach an sich bekannten Aufarbeitungsmethoden.

Die Erfindung betrifft somit:

1. Die Verbindung der Formel I

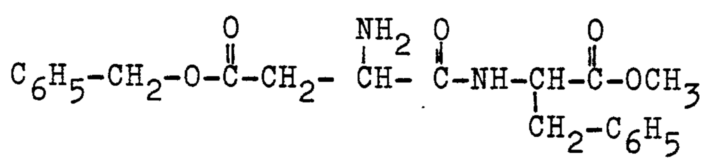

I

2. Das Verfahren zur Herstellung der Verbindung der Formel I,

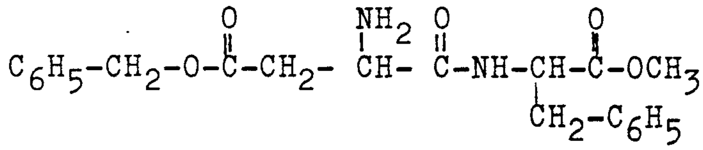

I

das dadurch gekennzeichnet ist, daß die Verbindung der Formel II,

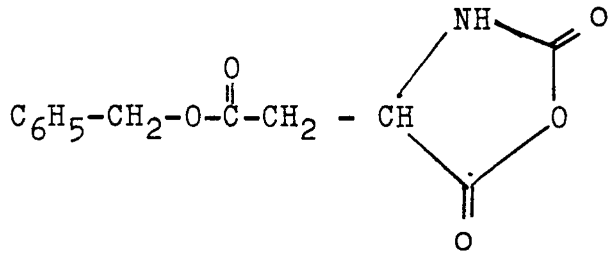

II

mit L-Phenylalaninmethylester in wäßriger Lösung bei pH 6,0 bis 8,5 umgesetzt wird.

3. Die Verwendung der Verbindung der Formel I zur Herstellung von Aspartam.

Die Verbindung der Formel II sollte zur Durchführung des obengenannten Verfahrens einen hohen, d.h. über 99 %igen Reinheitsgrad aufweisen. Sie kann am besten nach dem Verfahren von Fuchs-Farthing durch Einwirkung von Phosgen auf 4-Benzyl-L-aspartat in Dioxan oder Tetrahydrofuran und anschließende Reinigung erhalten wer den [Advanced Protein Chemistry 13, S.24335 (1958) und J.Am.Chem.Soc. 82, S 2269 (1960) ]. 4-Benzyl-L-aspartat ist wiederum, wie beispielsweise in der Patentanmeldung P 35 20 8082 vorgeschlagen, durch selektive Hydrierung von L-Asparaginsäure-dibenzyl-ester-tosylat leicht zugänglich.

Die Verbindung der Formel II kann als Pulver verwendet werden. Günstiger ist jedoch der Einsatz von Lösungen oder Dispersionen der Verbindung in nichtprotischen organischen Lösungsmitteln, wobei die Substanz in der 2 bis 40 fachen Menge, bevorzugt 5 bis 20 fachen Menge, des Lösungsmittels, bezogen auf das Gewicht, aufgenommen wird. Geeignete Lösungsmittel sind z.B. Toluol, Ethylacetat und bevorzugt Methylenchlorid.

Der L-Phenylalaninmethylester kann in seiner freien Form eingesetzt werden. Da die Verbindung jedoch instabil ist und selbst bei Temperaturen unter 0° C allmählich in 3,6-Di-benzyl-2,5-di-keto-piperazin übergeht, ist es vorteilhaft, ihre chemisch stabilen Salze in Kombination mit Basen einzusetzen. Hierzu sind beispielweise die anorganischen Basen geeignet, wie Natrium-oder Kaliumhydroxid, bzw. bevorzugt Natrium-oder Kaliumcarbonat oder Kaliumbicarbonat und besonders bevorzugt Natriumbicarbonat. Das entstehende Alkalisalz kann dann später, zusammen mit überschüssigem L-Phenylalaninmethylester, beim Abtrennen der wäßrigen Phase entfernt werden. Die Verwendung des L-Phenylalaninmethylesterhydrochlorids ist ebenfalls bevorzugt. Der L-Phenylalaninmethylester wird in freier Form bzw. in Form seiner stabilen Salze als wäßrige Lösung eingesetzt. Der Ester kann in der 2 bis 20 fachen Menge, bevorzugt der 4 bis 10 fachen Menge, Wasser aufgenommen werden.

Der D-Phenylalaninmethylester bzw. das Racemat kann unter den gleichen Bedingungen eingesetzt werden wie der L-Phenylalaninmethylester.

Da das Reaktionsprodukt, die Verbindung der Formel I, mit der Verbindung der Formel II zu einem unerwünschten Tripeptid vom Typ Phe (OMe)-Asp(OBz)-Asp(OBz) weiterreagieren kann, wirkt die Anwendung eines Überschusses von L-Phenylalaninmethylester ausbeutesteigernd. Es empfiehlt sich daher der Einsatz von 1,5 -20 mol, bevorzugt 2,2 -6 mol L-Phenylalaninmethylester pro Mol der Verbindung der Formel II.

Der Ph-Wert der Reaktionsmischung sollte bei der Zugabe der Verbindung der Formel II zwischen 6,0 und 8,5, bevorzugt zwischen 6,5 und 8,2, liegen. Er kann mit den oben aufgeführten anorganischen Basen eingestellt werden.

Das Anhydrid der Formel II wird dem Reaktionsgemisch bevorzugt über einen Zeitraum von 3-30 Minuten zugegeben, wobei 120 Minuten zweckmäßig nicht überschritten werden sollen. Nach beendeter Zugabe der Verbindung der Formel II fällt der pH-Wert im allgemeinen um ca. 0,1 bis 1,5 Einheiten ab. Günstigste Reaktionstemperaturen liegen zwischen 0° und 30°C, bevorzugt zwischen 5° und 15°C. Nachrührzeiten zwischen 1 und 30 Minuten, bevorzugt zwischen 3 und 10 Minuten, sind zweckmäßig.

Zur Stabilisierung des entstandenen Benzylaspartams während der anschließenden Aufarbeitung werden nach dem Nachrühren pH-Werte zwischen 6,2 und 2,0 bevorzugt 6,0 und 3,0 eingestellt. Es werden soviel Mol einer anorganischen Säure, bevorzugt Salzsäure, zugesetzt, daß mindestens drei Viertel des Überschusses von Phenylalaninmethylester neutralisiert werden bzw. höchstens 110 % der Säure, bezogen auf die Molmenge des ingesamt eingesetzten L-Phenylalaninmethylesters, zugegeben werden.

Alternativ kann das gebildete Benzylaspartam schnell aus der aggressiven alkalischen Wasserphase entfernt werden, indem der wäßrigen Lösung des L-Phenylalaninmethylester schon vor der Anhydridzugabe ein mit Wasser nicht mischbares Lösungsmittel, wie z.B. Toluol, Ethylacetat oder bevorzugt Methylenchlorid, in 0,5 -20 facher Menge, bevorzugt 2 bis 4 facher Menge, zugesetzt wird. Zweckmäßig wird hier das gleiche Lösungsmittel gewählt, in dem auch das Anhydrid suspendiert wird. Esterhydrolysen sowie die Bildung von 3-Benzyl-6-(Benzyloxy-carbonyl)-methyl-2,5-diketo-piperazin werden auf diese Weise verhindert.

Die Art der Abtrennung des Überschusses von L-Phenylalaninmethylester vom gebildeten Reaktionsprodukt ist abhängig von dem evtl. vorher gewählten Lösungsmittel. Bei der Verwendung von z.B. Methylenchlorid empfiehlt sich zunächst nur die Einstellung eines pH-Werts von ca. 5 bis 6, weil dann nach dem Ausschütteln und der Abtrennung der Wasserphase das Reaktionsprodukt der Formel I quantitativ zusammen mit nur noch ca. einem Fünftel der ursprünglichen Menge an L-Phenylalaninmethylester in der organischen Phase verbleibt. Die Hauptmenge des Überschusses von L-Phenylalaninmethylester befindet sich in der Wasserphase als Hydrochlorid und kann daraus durch Freisetzung des basischen Ester und dessen Extraktion mit einem organischen Lösungsmittel, wie z.B. Methylenchlorid und ggf. Überführen in das stabile Hydrochlorid wiedergewonnen werden.

Zur vollständigen Abtrennung von L-Phenylalaninmethylester aus der organischen Phase wird diese nochmals mit Wasser ausgeschüttelt und ggf. dabei z.B. mit Salzsäure ein etwas niedrigerer pH-Wert von ca. 4 -5,0 eingestellt. Durch diese pH-Wert-Erniedrigung geht zwar auch ein geringer Teil des Reaktionsprodukts in die Wasserphase über, dieser vorübergehende Ausbeuteverlust kann jedoch durch den Einsatz dieses wäßrigen Extrakts an Stelle von Wasser beim Ausschütteln der organischen Phase des nächsten gleichartigen Ansatzes vollkommen ausgeglichen werden.

Die derart gereinigte organische Phase, die praktisch nur noch das Reaktionsprodukt der allgemeinen Formel I und Chlorwasserstoff enthält, wird z.B. mit Molekularsieb getrocknet. Das Lösungmittel wird anschließend unter möglichst milden Bedingungen, d.h. bei maximal ca. 40°C, abdestilliert. Zur Verhinderung der Bildung des asymmetrischen 2,5-Diketopiperazins, das sich aus dem Reaktionsprodukt der Formel I im alkalischen Medium leicht bilden kann, sollten beim Abdestillieren des Lösungsmittels 0,01 -1 mol, bevorzugt 0,05 bis 0,5 mol einer Säure, bevorzugt Essigsäure oder Salzsäure, zugegen sein.

Ein chlorwasserstofffreies Reaktionsprodukt der Formel I kann durch Behandlung der Lösungen der chlorwasserstoffhaltigen Produkte in organischen Lösungsmitteln mit wäßrigen Lösungen von basischen Alkaliverbindungen, wie z.B. Soda oder Natriumbicarbonat, Trocknung der organischen Phase und Abdestillieren des Lösungsmittels erhalten werden und sollte möglichst bald weiterverarbeitet werden.

Die Erfindung wird durch die folgenden Beispiele näher erläutert.

## Beispiele

Die Reaktionskontrollen erfolgen durch HPLC und $^1$H-NMR-Spektroskopie.

Die in den Beispielen aufgeführten HPLC-Analysen werden an "reversed phase"-Kieselgel, das mit Alkylresten mit 8 C-Atomen silyliert ist (RP 8), und einem Fließsystem aus Methanol und Wasser (1:1,v:v) bei einem pH-Wert von 2,8 durchgeführt.

## Beispiel 1

In einen 1-Liter-Vierhalskolben werden 43,1 g (200 mmol) L-Phenylalaninmethylester-hydrochlorid, 200 g Wasser, 400 g Methylenchlorid und 16,8 g (200 mmol) Natriumbicarbonat eingewogen und auf 10°C abgekühlt. Dabei stellt sich ein pH-Wert von 7 bis 8 ein, der mit Merck-Universalindikator-Papier gemessen wird.

Innerhalb von 10 Minuten wird dem Gemisch bei 10°C die im Ultraschallbad stabilisierte Suspension von 12,5 g (50 mmol) kristallinem 4-Benzyl-L-aspartat-N-carbonsäure-anhydrid (Fp. 128 -130°C) in 100 g Methylenchlorid zugefügt. Nach 5 Minuten Nachrühren werden 35 ml (140 mmol) 4 molarer Salzsäure zugesetzt wodurch man einen pH-Wert von ca. 5 bis 6 erhält.

Nach dem Abtrennen der wäßrigen Phase 1, welche gemäß HPLC-Analyse die Hauptmenge des überschüssigen L-Phenylalaninmethylester-hydrochlorids, aber praktisch kein Benzylaspartam enthält, wird die Methylenchlorid-Phase 1 mit 200 g Wasser und 5 ml 4 molarer Salzsäure nochmals extrahiert. Dabei gehen der Rest des L-Phenylalaninmethylester und ca. 15 % des Benzylaspartam als Hydrochlorid in die wäßrige Phase 2 über, während die Hauptmenge des Reaktionsprodukts in der organischen Phase 2 verbleibt. Nach dem Trocknen der Methylenchlorid-Phase 2 mit 10 g Molekularsieb (4 nm, 2 mm Perlendurchmesser, Fa. Riedel der Haën) und dem Abdestillieren des Methylenchlorids bei 25°C im milden Vakuum hinterbleiben 17,8 g Rückstand. Er enthält (gemäß HPLC) 98,5 % Benzylaspartam, wobei ca. 16 % als Hydrochlorid vorliegen.

Beispiel 2

Man verfährt gemäß Beispiel 1, schüttelt jedoch die Methylenchlorid-Phase 1 mit 206 g der wäßrigen Phase 2 von Beispiel 1 aus, unter Zusatz von 5 ml 4 molarer HCl.

Es resultieren 19,3 g Benzylaspartam (97,4 % d.Th.), von dem ca. 16 % als Hydrochlorid vorliegen.

Beispiel 3

Man verfährt gemäß Beispiel 2. Die Methylenchlorid-Phase 2 wird jedoch mit der Lösung von 3,36 g - (40 mmol) Natriumcarbonat in 200 g Wasser ausgeschüttelt. Die so erhaltene organische Phase 3 wird mit 10 g Molekularsieb getrocknet. Nach dem Abdestillieren des Methylenchlorids hinterbleibt ein farbloses Öl, das kein ionogenes Chlor enthält. Im HPLC-Chromatogramm liegt nur der Benzylaspartam-Peak vor. Das $^1$H NMR-Spektrum stimmt mit der Theorie überein.

Ausbeute: 19,0 g (99.0 % d.Th.).

Brechungsindex: $n_D^{20}$ = 1,5456

Drehwert: $[\alpha]_D^{20}$ = + 11,6 (in Methanol)

Beispiel 4

Man verfährt gemäß Beispiel 2. Es werden jedoch statt 16,8 g (200 mmol) Natriumbicarbonat 10,6 g - (100 mmol) Soda als Base eingesetzt.

Man erhält gemäß HPLC 19.4 g reines Benzylaspartam, von dem ca. 21 % als Hydrochlorid vorliegen.

**Ansprüche**

1. Die Verbindung der Formel I,

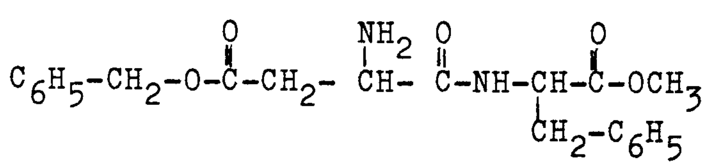

I,

2. Verfahren zur Herstellung der Verbindung der Formel I,

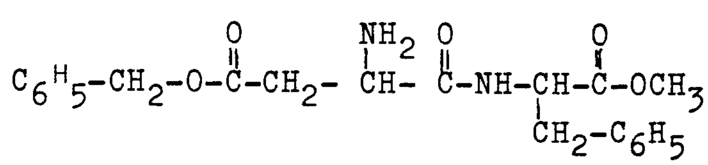

I

dadurch gekennzeichnet, daß die Verbindung der Formel II,

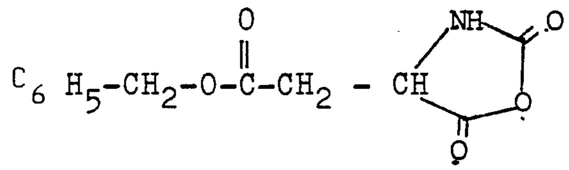

II

mit Phenylalaninmethylester in wäßriger Lösung bei pHWerten von 6,0 bis 8,5 umgesetzt wird.

3. Verfahren nach Anspruch 2, dadurch gekennzeichnet, daß die Reaktion bei pH-Werten von 6,5 bis 8,2 durchgeführt wird.

4. Verfahren nach Anspruch 2 oder 3, dadurch gekennzeichnet, daß mit 1 mol der Verbindung der Formel II 1,5 bis 20 mol L-Phenylalaninmethylester umgesetzt werden.

5. Verfahren nach Anspruch 4, dadurch gekennzeichnet, daß 2,2 bis 5 mol L-Phenylalaninmethylester eingesetzt werden.

6. Verfahren nach einem oder mehreren der Ansprüche 2 bis 5, dadurch gekennzeichnet, daß die Verbindung der Formel II als Lösung oder Dispersion in nichtprotischen organischen Lösungsmitteln eingesetzt wird.

7. Verfahren nach einem oder mehreren der Ansprüche 1 bis 6, dadurch gekennzeichnet, daß der L-Phenylalaninmethylester in Form der chemisch stabilen Salze eingesetzt wird.

8. Verwendung der Verbindung der Formel I

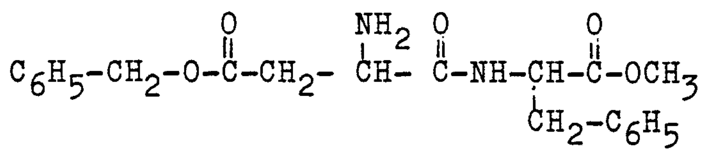

I

zur Herstellung von d-L-Aspartyl-L-phenylalaninmethylester.

9. Verwendung der nach einem oder mehreren der Ansprüche 2 bis 7 erhältlichen Verbindung der Formel I zur Herstellung von d-L-Aspartyl-L-phenylalaninmethylester.